# EUROPEAN PATENT APPLICATION

(11) **EP 4 835 901 A1**
(43) Date of publication of application: **04.11.2026**
(21) Application number: 26156923.0
(22) Date of filing: 06.02.2026
(51) Int. Cl.: G16H 10/20, G16H 20/10, G16H 40/67, G16H 50/70, G16H 70/40, G16H 80/00, G06N 3/045

(54) **SYSTEM AND METHOD FOR AUTOMATIC REPORTING OF ADVERSE DRUG REACTIONS**

(30) Priority: 30.04.2025 US 202519195147
(71) Applicant: Accenture Global Solutions Limited, Dublin 4 (IE)
(72) Inventor: SINGH, Prashant, 400607 Thane (IN); RANE, Parag Bapu, 400601 Thane West (IN); RAMCHANDANI, Sumit Jagdish, 400607 Thane West (IN); CHAUDHARY, Mukesh Kumar, 560066 Bangalore (IN); JUNEJA, Saurabh, 110075 New Delhi (IN); GHOSH, Vaswati, 400072 Mumbai (IN)
(74) Representative: Müller-Boré & Partner Patentanwälte PartG mbB

(57) **Abstract**

Method, system, and non-transitory computer-readable medium for automatic reporting of an adverse drug reaction are disclosed. In an aspect, data associated with a plurality of agents of an agent tree is retrieved. Further, task data of each agent of the plurality of agents is retrieved. Furthermore, the retrieved data associated with the plurality of agents and the task data of each agent of the plurality of agents are analyzed for refining task prompts and delegation of one or more tasks to an agent of the plurality of agents. Based upon the analyzing, a list of optimum parameters and interaction of the agent with other agent of the plurality of agents is generated. Also, using the list of optimum parameters, one or more backstories are generated for training the agent of the plurality of agents. For example, the agent is trained for automatic reporting of the adverse drug reaction.

## Description

### TECHNICAL FIELD

Various examples described herein relate generally to a method, system, and non-transitory computer readable medium for automatic reporting of adverse drug reactions. Specifically, disclosed examples are directed to techniques for automatic reporting of the adverse drug reactions using multiple large language models (LLMs).

### BACKGROUND

Adverse drug reactions (ADRs) refer to harmful or unintended reactions that occur after administration of a medication. These reactions can vary in terms of severity, ranging from mild symptoms to life-threatening conditions. The ADRs are significant public health concern, contributing to increased morbidity, mortality, and healthcare costs. Given the widespread use of pharmaceuticals across diverse populations, it is critical to monitor, detect, and prevent drug-related adverse effects in order to enhance patient safety and improve therapeutic outcomes. The ability to quickly identify, report, and address the ADRs is essential for ensuring patient safety and improving efficacy of pharmaceutical products.

### SUMMARY

Implementations of the present disclosure are generally directed to reporting of adverse drug reactions. More particularly, implementations of the present disclosure are directed to techniques for automatic reporting of the adverse drug reactions using multiple large language models (LLMs).

In some examples, aspects of the subject matter described herein provide a computer-implemented method for automatic reporting of the adverse drug reactions. The method may include retrieving, by one or more processors of one or more computing devices, data associated with a plurality of agents of an agent tree. Further, the method may include retrieving, by the one or more processors of the one or more computing devices, task data of each agent of the plurality of agents. Furthermore, the method may include analyzing, by the one or more processors of the one or more computing devices, the retrieved data associated with the plurality of agents and the task data of each agent of the plurality of agents for refining task prompts and delegation of one or more tasks to an agent of the plurality of agents. Based upon the analyzing, the method may include generating, by the one or more processors of the one or more computing devices, a list of optimum parameters and interaction of the agent with other agent of the plurality of agents. Also, the method may include generating, by the one or more processors of the one or more computing devices, using the list of optimum parameters, one or more backstories for training the agent of the plurality of agents, wherein the agent is trained for automatic reporting of the adverse drug reaction.

The present disclosure further describes a system for implementing the method provided herein. The present disclosure also describes one or more non-transitory computer-readable media coupled to the one or more processors of the one or more computing devices and having instructions stored thereon which, when executed by the one or more processors, cause the one or more processors to perform operations in accordance with the method described herein.

It is appreciated that method in accordance with the present disclosure can include any combination of the aspects and features described herein. That is, the method in accordance with the present disclosure is not limited to the combinations of aspects and features specifically described herein but also include any combination of the aspects and features provided.

The details of one or more implementations of the present disclosure are set forth in the accompanying drawings and the description below. Other features and advantages of the present disclosure will be apparent from the description and drawings, and from the claims.

### BRIEF DESCRIPTION OF THE DRAWINGS

Various examples in accordance with the present disclosure will be described with reference to the drawings, in which:
FIG. 1 depicts an example environment that may be used to execute implementations of the present disclosure.
FIG. 2 depicts an example architecture of an adverse drug reaction (ADR) reporting system, in accordance with implementations of the present disclosure.
FIG. 3 depicts an example schematic diagram illustrating various components of a multi-agent supervisor-worker large language model (LLM) framework, in accordance with implementations of the present disclosure.
FIG. 4 depicts an example method illustrating interactions between supervisor agents and corresponding worker agents while performing various tasks, in accordance with implementations of the present disclosure.
FIG. 5 depicts an example architecture illustrating a framework for adverse event data collection, in accordance with implementations of the present disclosure.
FIGS. 6A and 6B are flow diagrams representing example methods for detecting Artificial Intelligence (AI) generated content and Bot generated content, respectively, in accordance with implementations of the present disclosure.
FIG. 7 is a flow diagram that represents an example method for automatic reporting of ADRs, in accordance with implementations of the present disclosure.
FIG. 8 depicts a block diagram of an example computer system that may be used to implement the method of automatic reporting of the adverse drug reactions, in accordance with implementations of the present disclosure.

Like reference numbers and designations in the various drawings indicate like elements.

### DETAILED DESCRIPTION

In the following description, various examples will be illustrated by way of example and not by way of limitation in the figures of the accompanying drawings. References to various examples in this disclosure are not necessarily to the same example, and such references mean at least one. While specific implementations and other details are discussed, it is to be understood that this is done for illustrative purposes only. A person skilled in the relevant art will recognize that other components and configurations may be used without departing from the scope and spirit of the claimed subject matter.

Reference to any "example" herein (e.g., "for example," "an example of," by way of example," or the like) are to be considered non-limiting examples regardless of whether expressly stated or not.

The terms used in this specification generally have their ordinary meanings in the art, within the context of the disclosure, and in the specific context where each term is used. Alternative language and synonyms may be used for any one or more of the terms discussed herein, and no special significance should be placed upon whether or not a term is elaborated or discussed herein. Synonyms for certain terms are provided. A recital of one or more synonyms does not exclude the use of other synonyms. The use of examples anywhere in this specification including examples of any terms discussed herein is illustrative only and is not intended to further limit the scope and meaning of the disclosure or of any exemplified term. Likewise, the disclosure is not limited to various examples given in this specification.

Without intent to limit the scope of the disclosure, examples of instruments, apparatus, methods, and their related results according to the examples of the present disclosure are given below. Note that titles or subtitles may be used in the examples for convenience of a reader, which in no way should limit the scope of the disclosure. Unless otherwise defined, technical and scientific terms used herein have the meaning as commonly understood by one of ordinary skill in the art to which this disclosure pertains. In the case of conflict, the present document, including definitions will control.

The term "comprising" when utilized means "including, but not necessarily limited to"; it specifically indicates open-ended inclusion or membership in the so-described combination, group, series, and the like.

The term "a" means "one or more" unless the context clearly indicates a single element.

"First," "second," etc., re labels to distinguish components or blocks of otherwise similar names but does not imply any sequence or numerical limitation.

"And/or" for two possibilities means either or both stated possibilities ("A and/or B" covers A alone, B alone, or both A and B take together), and when present with three or more stated possibilities means any individual possibility alone, all possibilities taken together, or some combination of possibilities that is less than all of the possibilities. The language in the format "at least one of A ... and N" where A through N are possibilities means "and/or" for the stated possibilities (e.g., at least one A, at least one N, at least one A and at least one N, etc.).

It should also be noted that in some alternative implementations, the functions/acts noted may occur out of the order noted in the figures. For example, two steps disclosed or shown in succession may in fact be executed substantially concurrently or may sometimes be executed in the reverse order, depending upon the functionality/acts involved.

Specific details are provided in the following description to provide a thorough understanding of examples. However, it will be understood by one of ordinary skill in the art that examples may be practiced without these specific details. For example, systems may be shown in block diagrams so as not to obscure the examples in unnecessary detail. In other instances, well-known processes, structures, and techniques may be shown without unnecessary detail in order to avoid obscuring example examples.

The specification and drawings are to be regarded in an illustrative rather than a restrictive sense. It will, however, be evident that various modifications and changes may be made thereunto without departing from the broader spirit and scope as set forth in the claims.

This disclosure should be interpreted according to the exemplary definitions provided below. In case of a contradiction between the definitions in the definitions section and other sections of this disclosure, this section should prevail. In case of a contradiction between the definitions in this section and a definition or a description in any other document, including in another document incorporated in this disclosure by reference, this section should prevail, even if the definition or the description in the other document is commonly accepted by a person of ordinary skill in the art.

Currently, adverse drug reaction (ADR) reporting is largely performed through spontaneous reporting systems, where healthcare professionals, patients, or pharmaceutical companies report suspected adverse reactions to regulatory authorities. While these systems are essential, they often suffer from limitations such as underreporting, inconsistent data quality, delayed reporting, and lack of comprehensive data integration from multiple sources. Further, the healthcare professionals may experience barriers to report ADRs, including time constraints, lack of awareness of reporting procedures, and concerns about legal implications. Also, the ADRs may go undetected from social media and other web channels. This can lead to incomplete data and inaccurate risk assessments.

Further, existing systems and technologies for ADR reporting are typically manual and require significant human input, leading to inefficiencies and potential delays in the identification of new safety concerns. With the increasing volume and complexity of healthcare data, including electronic health records, social media, and wearable devices, it becomes challenging to efficiently analyze and extract relevant information for pharmacovigilance purposes.

There is also a need for better integration and analysis of the data to identify emerging safety signals with background noise and to enhance decision-making for regulatory agencies, healthcare providers, and pharmaceutical companies. Thus, there is a need for an improved method, system, or device to facilitate the timely, accurate, and efficient reporting of the ADRs.

Implementations of the present disclosure may automatically report the ADRs using multiple large language models (LLMs) based agentic framework. The present disclosure utilizes different groups of agents (e.g., the LLMs) in the agentic framework for performing various tasks in a pharmacovigilance process and automatically report the ADRs. Therefore, the implementations of the present disclosure for monitoring the ADRs provides valuable data that can guide future drug design, helping organizations in dveloping safer, more effective medications.

Leveraging real-world evidence (RWE) from ADR databases, the present disclosure may allow the organizations to fine-tune existing treatments and improve drug safety over time. Also, ensuring drug safety can increase a drug's lifecycle value by reducing a risk of withdrawal or restriction due to safety concerns. In turn, safer drugs may have longer market life, translating to sustained revenue.

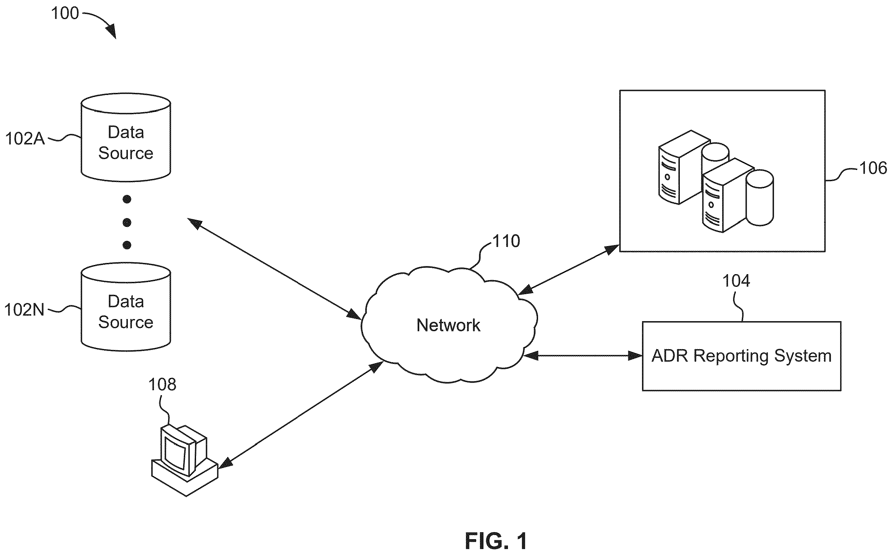

FIG. 1 depicts an example environment 100 that may be used to execute implementations of the present disclosure. The example environment 100, shown in FIG. 1, includes data sources 102A-N, an ADR reporting system 104, a storage device 106 and a user device 108. For simplicity, a single user device 108 is depicted in FIG. 1, however it should be noted that the example environment 100 may include one or more user devices. The data sources 102A-N, the ADR reporting system 104, the storage device 106 and the user device 108 may communicate with each other using a network 110. In some examples, the network 110 may include a Local Area Network (LAN), a Wide Area Network (WAN), the Internet, or a combination thereof. In some examples, the network 110 may be accessed over a wired and/or a wireless communication link.

The plurality of data sources 102A-N may include communication devices and/or computing devices that includes data associated with reports received from patients or healthcare providers, clinical research associates obtained during clinical trials, literature review specialists. The plurality of data sources 102A-N may include a server such as a web server, a database server, a host server, a proxy server, a virtual server (e.g., executing on a computing hardware), or a server in a cloud computing system.

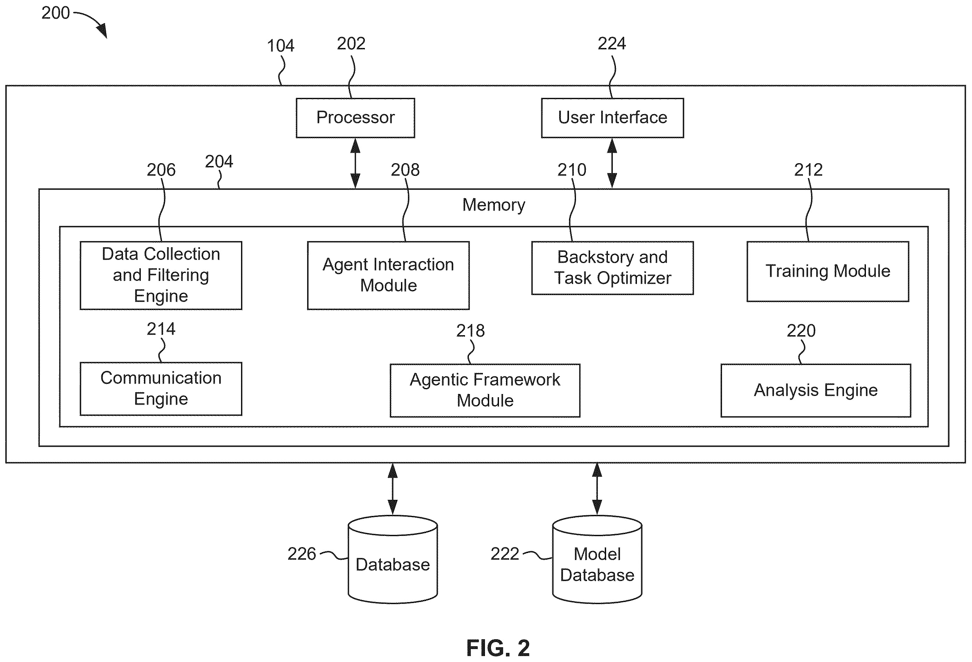

The ADR reporting system 104 is a computing device or system that receives or obtains the data from the plurality of data sources 102A-N. The ADR reporting system 104 may then process and store the data in the storage device 106. In some examples, the ADR reporting system 104 may include internal or external servers, quantum computers, desktops, laptops, smartphones, tablets, and/or the like. It is contemplated that implementations of the present disclosure may be realized with any appropriate type of computing device or computing platform. In some examples, the ADR reporting system 104 may display one or more Graphical User Interfaces (GUIs) that enable the user of the user device 108 to interact with a computing platform executing the ADR reporting. Examples of the computing platform may include content delivery platforms, multimedia-based platforms, and/or the like. Interacting with the computing platform may include providing feedback during the process of ADR reporting. For example, the ADR reporting system 104 is described in more detail with reference to FIG. 2.

While only one ADR reporting system 104 is shown in FIG. 1, there may be more than one ADR reporting system 104, and each of the ADR reporting system 104 includes at least one server system. In some examples, the server system hosts one or more computer implemented services that users can interact with by using the user device 108. For example, components of enterprise systems and applications can be hosted on one or more of the ADR reporting system 104. In some examples, the ADR reporting system 104 can be provided as an on-premises system that is operated by an enterprise or a third-party taking part in cross-platform interactions and data management. In some examples, the ADR reporting system 104 can be provided as an off-premises system (e.g., cloud or on-demand) that is operated by an enterprise or a third-party on behalf of an enterprise.

In some examples, the user device 108 may include computer executable applications executed thereon. The user device 108 may include a web browser application executed thereon, which can be used to display one or more web pages of applications executing on the ADR reporting system 104. In some examples, the user device 108 can display one or more GUIs that enable the respective the users to interact with the ADR reporting system 104. In accordance with implementations of the present disclosure, the ADR reporting system 104 may host enterprise applications or systems that require data sharing and data privacy.

In some implementations, the ADR reporting system 104 can be implemented in a cloud environment. The ADR reporting system 104 includes at least one server system (or server). In the example of FIG. 1, the ADR reporting system 104 can include various forms of servers including, but not limited to, a web server, an application server, a proxy server, a network server, and/or a server pool. In general, server systems accept requests for application services and provide such services to any number of client devices.

Further, the storage device 106 may include any standalone server or any type of computing device that are part of a cloud computing environment for storing data that is ingested by processing the input data. Various examples depicting automatic reporting of the ADRs are described in detail in conjunction with FIGS. 2-6.

FIG. 2 depicts an example architecture 200 of the ADR reporting system 104, in accordance with implementations of the present disclosure. As depicted in FIG. 2, the ADR reporting system 104 is communicatively coupled with a database 226 (e.g., the storage device 106) and a model database 222. For example, the database 226 can be a client database or a metadata database that is storing information related to the process of reporting ADRs, tools and the like. In some examples, the model database 222 may include LLMs, Generative Artificial Intelligence (GAI) models, foundation models, and/or the like. In an implementation, the LLMs may include pre-trained LLMs or generated LLMs. The pre-trained LLMs may be general-purpose GAI models like large deep learning neural networks, which may be trained using a broad range of generalized and unlabeled training data to perform one or more tasks, such as, human computer interactions (e.g., question and answering), automating process execution, process planning, generating step-by-step procedures for the process execution, performing data analysis, and/or the like. While implementations of the present disclosure are described in further detail herein with non-limiting reference to the LLMs, it is contemplated that implementations of the present disclosure may be realized using any appropriate foundation models or Machine Learning (ML) models, or Artificial Intelligence (AI) models. In some examples, the LLMs can be on-premises LLMs, or cloud-based LLMs.

As depicted in FIG. 2, the ADR reporting system 104 includes a processor 202, a memory 204 and a user interface 224. The ADR reporting system 104 may also include other components such as communication interfaces, Input/Output (I/O) devices, and so on (not shown in FIG. 2). The processor 202 may include one or more processors. Examples of the one or more processors may include, but not limited to, microprocessors, microcomputers, microcontrollers, digital signal processors, central processing units, state machines, logic circuits, Application Specific Integrated Circuits (ASICs), Field Programmable Gate Arrays (FPGAs), and/or any devices that manipulate data or signals based on operational instructions. Among other capabilities, the processor 202 may be programmed to execute computer-readable instructions stored in the memory 204 (also referenced herein as non-transitory computer-readable medium (NTCRM)) for performing operations according to the present disclosure. The memory 204 may be non-transitory or non-volatile medium, such as a magnetic disk or solid-state non-volatile memory or volatile medium such as Random Access Memory (RAM), and/or the like.

The ADR reporting system 104 further includes a data collection and filtering engine 206, an agent interaction module 208, a backstory and task optimizer 210, a training module 212, a communication engine 214, an agentic framework module 218, and an analysis engine 220 as depicted in FIG. 2. The data collection and filtering engine 206, the agent interaction module 208, the backstory and task optimizer 210, the training module 212, the communication engine 214, the agentic framework module 218, and the analysis engine 220 may be stored in the memory 204 and provided as a downloadable library including the computer-readable instructions. The data collection and filtering engine 206, the agent interaction module 208, the backstory and task optimizer 210, the training module 212, the communication engine 214, the agentic framework module 218, and the analysis engine 220 may be executed by the processor 202 communicatively coupled with the memory 204 for automatically reporting the ADRs. In some implementations, data collection and filtering engine 206, the agent interaction module 208, the backstory and task optimizer 210, the training module 212, the communication engine 214, the agentic framework module 218, and the analysis engine 220 may leverage multiple customized logics for performing its intended functions. In some examples, multiple customized logics may include Generative Artificial Intelligence (Gen AI) models, Deep Learning (DL) models, deep neural networks, and/or the like.

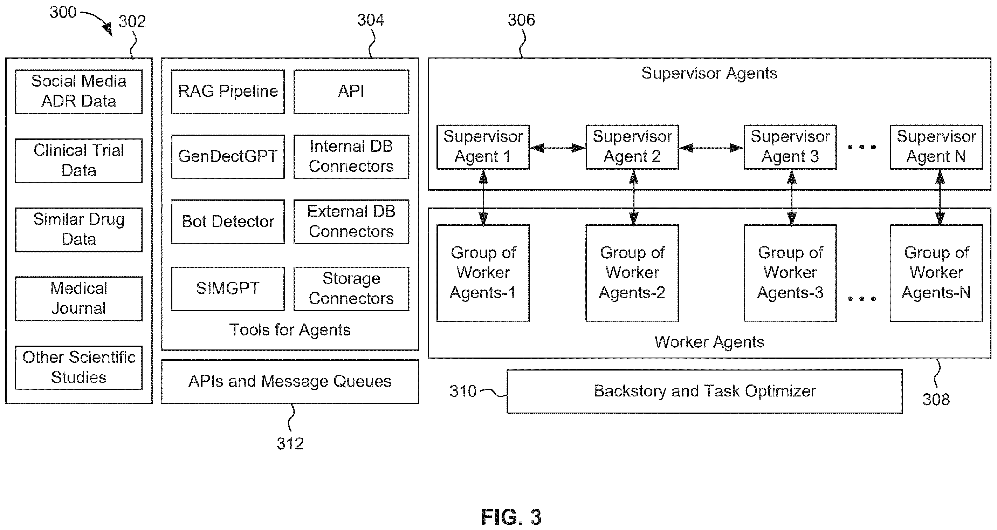

Generally, pharmacovigilance (PV) process ensures the safety of medicines and medical products by monitoring, detecting, assessing, and preventing adverse effects or any drug-related problems. In an example implementation, the ADR reporting system 104 performs the pharmacovigilance process by performing various steps, such as adverse event (AE) data collection, case intake and triage, case processing, signal detection and analysis, risk management, regulatory reporting, continuous benefit-risk evaluation, communication of safety information, and audit and compliance. In this example implementation, the agentic framework module 218 creates an agent tree including a group of agents or a plurality of agents for each step in the pharmacovigilance process. For example, the group of agents includes one or more supervisor agents, and one or more working agents associated with each supervisor agent of the one or more supervisor agents. Each of the one or more supervisor agents may manage overall workflow, assigns tasks to the worker agents, validates results, stores output and communicate to next supervisor agent in a queue to initialize tasks with their group of agents and so on. The worker agents are specialized in specific tasks such as data extraction, filtering, validation, enrichment and regulatory document creation. The worker agents perform tasks, and results are communicated back to the corresponding supervisor agent. In this example, the one or more supervisor agents and corresponding worker agents in a group includes the LLMs stored in the model database 222 and communicate with each other using a message-queue via the communication engine 214. An example schematic diagram illustrating various components of a multi-agent supervisor-worker LLM framework 300 is explained in more detail with reference to FIG. 3. In some examples, a supervisor agent can be an advanced LLM (e.g., GPT-4 or similar) that can manage multi-tasking, prioritize tasks, and generate insights from aggregated data and worker agents are multiple smaller instances of the LLM tuned for specific tasks.

Further, the data collection and filtering engine 206 may retrieve data associated with a plurality of agents of an agent tree. The plurality of agents may include the one or more supervisor agents 306, and the one or more working agents 308 associated with each supervisor agent of the one or more supervisor agents 306. For example, the data associated with the plurality of agents may include persona data of an agent, purpose and alignment of the agent with overall goal of the ADR reporting system 104, a LLM that is used (e.g., GPT-4, Claude, and the like) and corresponding configuration data of the LLM, a list of one or more internal or external tools or applications and corresponding API integrated with the agent, a list of document functions or APIs invoked by the LLM during execution, and/or one or more operational parameters. In this example, the data collection and filtering engine 206 may retrieve the persona data of an agent that define an agent's role, capabilities, and relationship within the framework 300 (e.g., a social media worker agent for scraping ADR data). In some examples, the one or more operational parameters may include a maximum iteration limit before returning to a best option, a maximum request per minute limit for requests, a maximum retry limit allowed upon failure, step callback functions or methods invoked after every iteration (e.g., progress tracking), storage (e.g., memory and cache) usage for intermediate data or results.

In this example, the list of one or more tools integrated with the agent includes one or more of a retrieval-augmented generation (RAG) pipeline, an application programming interface (API), a genetic detection generative pre-trained transformer (GenDectGPT), internal database (DB) connectors, a Bot detector, external DB connectors, a simulated interactive model using GPT (SIMGPT), storage connectors, social media scraping tools, web crawlers and the like.

Further, the data collection and filtering engine 206 may retrieve task data of each agent of the plurality of agents. For example, the task data may include detailed description of tasks assigned to each agent, a goal or deliverable or expected output associated with the task assigned to each agent, a list of one or more functions to be called upon completion of the task assigned to the agent or upon step execution, and/or a asynchronous call flag identifying whether the task assigned to the agent supports asynchronous operations.

Furthermore, the analysis engine 220 analyzes the retrieved data associated with the plurality of agents and the task data of each agent of the plurality of agents for refining task prompts and delegation of one or more tasks to an agent of the plurality of agents. In an example implementation, to analyze the retrieved data associated with the plurality of agents and the task data of each agent of the plurality of agents, the analysis engine 220 may identify a communication type of communication of the agent with the other agent. The communication type may be a sequential communication, a hierarchical communication, and/or a consensual communication. For example, the sequential communication may refer to a communication where an agent completes assigned task before next agent starts executing. The hierarchical communication may refer to a communication where a supervisor agent delegates tasks to corresponding worker agents. The consensual communication may refer to a communication where agents collaborate and collectively decide on task execution (e.g., multi-agent consensus models).

Based upon the identified communication type, the analysis engine 220 may delegate data collection task for generating one or more backstories to the agent of the plurality of agents. The data collection task may include optimizing a prompt or a task prompt, extracting data using the optimized prompt, and/or cleaning the extracted data. In an aspect, the analysis engine 220 may gather relevant and usable data for backstory generation. For example, a prompt can be: "Scrape recent tweets mentioning 'nausea' and 'headache' in a context of antidepressants." The worker agents use the prompt to search, extract, and clean the data. For each agent, the analysis engine 220 may identify expected steps to achieve the task and define how the agent's backstory (persona, purpose) aligns with narrative of the task and overall process. Further, the analysis engine 220 may define an exit criteria and task delegation. The exit criteria may include conditions to mark a task as complete (e.g., 80% data accuracy or successful API call) and the task delegation may specify conditions for task reassignment (e.g., retry limit exceeded, communication failure and the like).

In this example implementation, the analysis engine 220 may generate an agent-level log, a task-level log, and/or a communication log. For example, the agent-level log may include execution time, resource utilization (memory or cache), a number of iterations, retries, requests made, output generated and corresponding quality assessment and the like. The task-level log may include task description, execution start and/or end times, callback triggers, asynchronous execution status and results. The communication log may include the communication type, messages exchanged between agents and corresponding timestamps.

Moreover, the analysis engine 220 may analyze the agent-level log, the task-level log, and/or the communication log and identify one or more of a bottleneck in agent communication, an opportunity for retry optimization, a task prompt refinement, and/or enhanced delegation-and-alignment strategies of an agent selection.

Based upon the analyzing, the agent interaction module 208 may generate a list of optimum parameters and interaction of the agent with other agent of the plurality of agents. For example, the optimum parameters may include node features, edge features, a target variable, training dataset, a loss function, validation dataset, prediction data, testing dataset, an error range and the like. In an example implementation, to generate the list of optimum parameters and interaction of the agent with the other agent of the plurality of agents, the agent interaction module 208 may generate a plurality of node features, a plurality of edge features, and/or the target variable. For example, the plurality of node features corresponds with features of the agent and the agent represent a node of a network graph. The plurality of edge features may correspond to vector representation of parameters including a maximum number of iterations, a maximum number of requests per minute, a maximum number of retry limits, a memory, a cache, a minimum number of retries as a dependent variable and/or a step callback.

Further, in this example implementation, the agent interaction module 208 may split data of each edge of the network graph into one or more of the training dataset, the validation dataset, and/or the test dataset. Also, to capture a local graph structure and node or edge features, the the agent interaction module 208 uses a combination of graph convolutional networks (GCNs), graph attention networks (GATs), and graph sample and aggregation (GraphSAGE). Furthermore, the agent interaction module 208 performs node feature aggregation by aggregating features from neighbouring nodes to capture the local graph structure. In addition, the agent interaction module 208 may perform edge feature construction by combining the node features and the edge features to create a unified representation for each edge.

In some examples, the training module 212 may train a model on the training dataset using gradient descent or another optimization algorithm. Further, the agent interaction module 208 monitors performance on the validation dataset to avoid overfitting. For example, a regression loss function such as mean squared error (MSE) or mean absolute error (MAE) is used to evaluate the performance. Furthermore, the agent interaction module 208 may use the trained model to predict a combination of a node and each edge in the test dataset. Furthermore, the agent interaction module 208 may determine an error range (z-score) by computing standard deviation of residuals (errors) on the validation set and calculating a confidence interval for each prediction using the standard deviation.

Using the list of optimum parameters, the backstory and task optimizer 210 may generate one or more backstories for training the agent of the plurality of agents. For example, the agent is trained for automatic reporting of the ADR. In an example implementation, for optimizing the backstories and task prompts, the backstory and task optimizer 210 may retrieve data at agent level in the agent tree. The data may include historical instructions or guidelines of the agents, task prompts that provides historical instructions or guidelines for agent tasks, a numerical weight: that is a combination of the optimum value of the minimum number of retries, an agent level number of edges and feedback from user interactions and an expected output from the task prompt.

In an example implementation, the backstory and task optimizer 210 may represent inputs as text1 with agent level backstory with persona details, text2 with agent task level-related prompt to perform a certain action, a minimum number of retries (e.g., 3), a number of connected edges (e.g., 4), human feedback score (e.g., 0 not expected and 1 is expected). Further, the backstory and task optimizer 210 may combine the inputs into a structured format for the model. For example,
text 1: <instructions> text 2: <context> Score: <vectore of numbers>

Furthermore, the backstory and task optimizer 210 may use the model with generative backstory architecture. Then, the backstory and task optimizer 210 modify the input embeddings to include the numerical weight as a conditioning feature. The numerical weight may influence the output as part of an expected reward of training using actor-critic method. Generally, the actor-critic method is a type of reinforcement learning (RL) algorithm that combines both value-based and policy-based approaches.

During the actor-critic method, where actor is a transformer model, the training module 212 generates text based on the input (text 1 + text 2). During training, the actor is updated to maximize the expected reward. The reward is in a reverse proportion of numerical weights (e.g., 1/ (minimum number of retries + agent edges count + human feedback flag) *100). Further, the critic is a separate neural network that estimates a value of the generated text using a text gradient. In some examples, to determine the text gradient, a loss function L(x) (e.g., a regression loss function) is defined, where x represents a combination of agent's backstory and text prompt. Then, it is evaluated to determine how effectively the generated text meets a predefined objective. Further, a partial derivative of the loss function e.g., a gradient of the loss is computed concerning token embeddings. Then, backpropagation is utilized through a language model or a machine learning model to propagate errors. Furthermore, chain rule differentiation is applied across embedding spaces to refine token-level adjustments. For example, for sequence-based tasks, such as language modelling, translation, or text generation, tokens represent smallest units of meaning, such as words, sub-words, or characters. The token-level adjustments can involve various operations or manipulations made at a level of the individual tokens during the model's training or inference process.

In addition, in this example implementation, the backstory and task optimizer 210 takes the generated text and input details as input and outputs a scalar value representing the expected reward. The training module 212 then trains the critic to minimize a difference between corresponding predicted value and an actual reward.

In some examples, use a proximal policy optimization (PPO) algorithm, an actor-critic method, to train the model. During the training, the training module 212 iteratively performs generating text using the actor, calculating the reward using a reward function, updating the actor and critic based on the reward. Further, the backstory and task optimizer 210 may generate the back stories and the task prompts from the trained model.

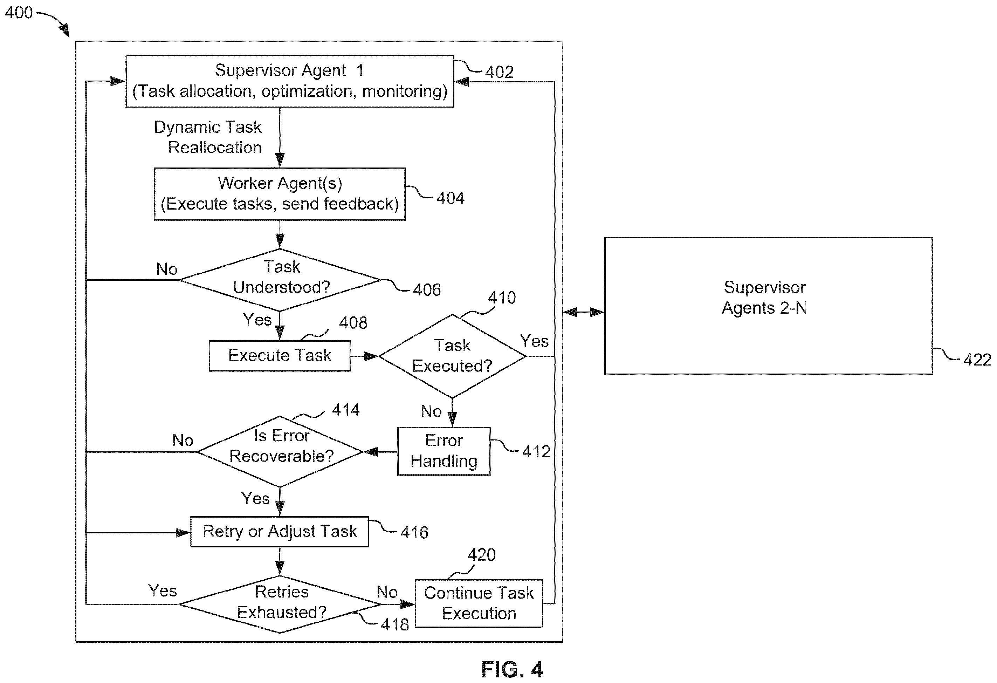

Referring now to FIG. 3, an example schematic diagram illustrating various components of a multi-agent supervisor-worker LLM framework 300, in accordance with implementations of the present disclosure. In an aspect, the components of the framework 300 can be recited in on-premises LLM framework and/or a cloud-based framework. In this aspect, the framework 300 includes input data 302 received from the plurality of data sources 102A-N. For example, the input data 302 may include social media ADR data, clinical trial data, similar drug data, medical journals and other scientific data received from one or more of webpages, social media, forums, blogs, audio/video platforms, image sources and the like. Further, the framework 300 includes a plurality of supervisor agents 306 and corresponding worker agents 308 for performing the steps involved in the pharmacovigilance process. An example process flow and interactions between supervisor agents and corresponding worker agents while performing various tasks is explained in more detail with reference to FIG. 4. Also, the plurality of supervisor agents 306 and corresponding worker agents 308 communicate with each other using APIs and message queues 312 via the communication engine 214. For example, the message queues 312 (e.g., Apache Kafka and the like) may facilitate communication between worker and supervisor agents for real-time processing. The supervisor agent uses task queues to assign scraping, filtering, processing, transforming and document generation tasks to the worker agents. In an aspect, the supervisor agent may use load balancers to distribute tasks evenly across the worker agents, ensuring the system 104 remains efficient. Also, end-to-end encryption for sensitive information flowing between Supervisor, Worker agents, and external databases is performed.

In an example implementation, as shown in FIG. 3, the framework 300 includes a group of agents including one or more supervisor agents and corresponding worker agents for performing each step in the pharmacovigilance process. For example, each of the steps, such as the AE data collection and Bot and AI generated content filtration, the case intake and triage, the case processing, the signal detection and analysis, the risk management, the regulatory reporting, the continuous benefit-risk evaluation, the communication of safety information, and the audit and compliance is performed using corresponding group of agents. Furthermore, the framework 300 may include a plurality of tools 304 to be used by the plurality of supervisor agents 306 and corresponding worker agents 308 for performing various tasks. For example, the plurality of tools 304 includes a RAG pipeline, an API, a GenDectGPT, internal database (DB) connectors, a Bot detector, external DB connectors, a SIMGPT, storage connectors and the like. Also, the plurality of supervisor agents 306 and corresponding worker agents 308 performs the steps involved in the pharmacovigilance process using a backstory and task optimizer 310 (e.g., the backstory and task optimizer 210).

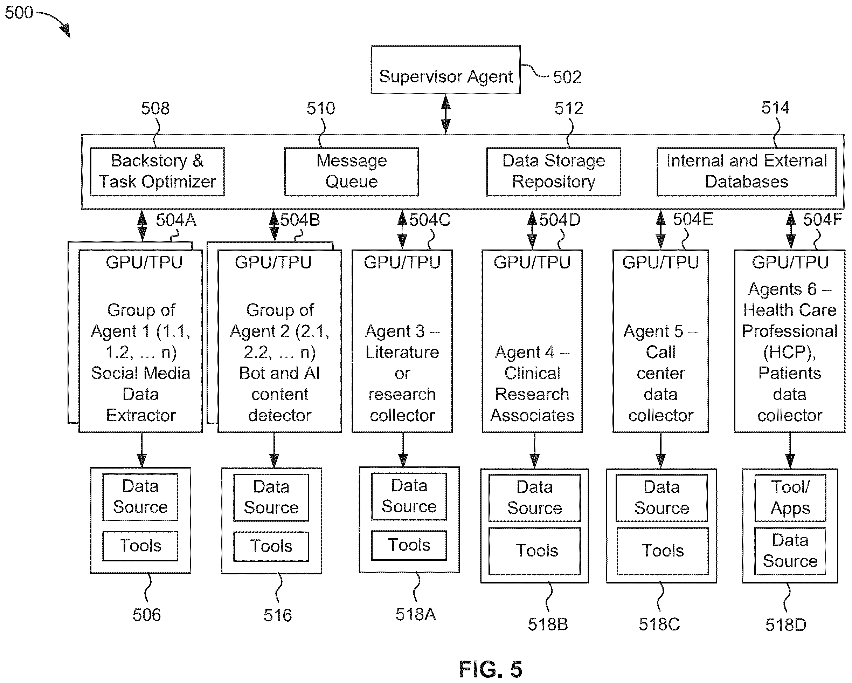

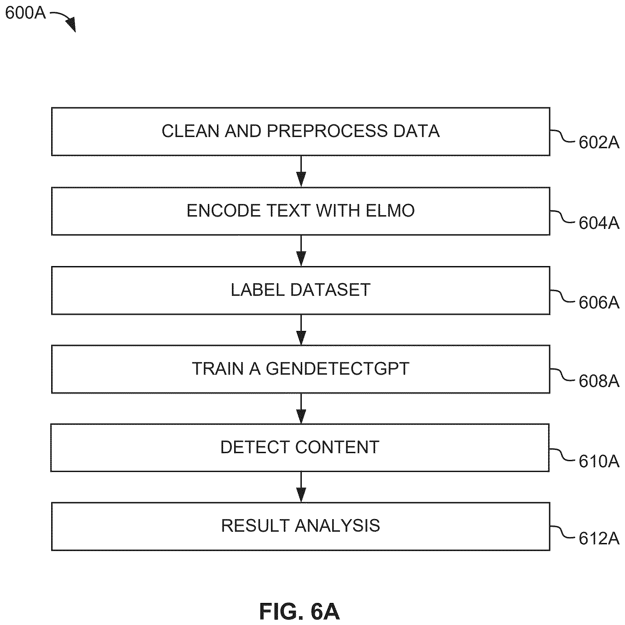

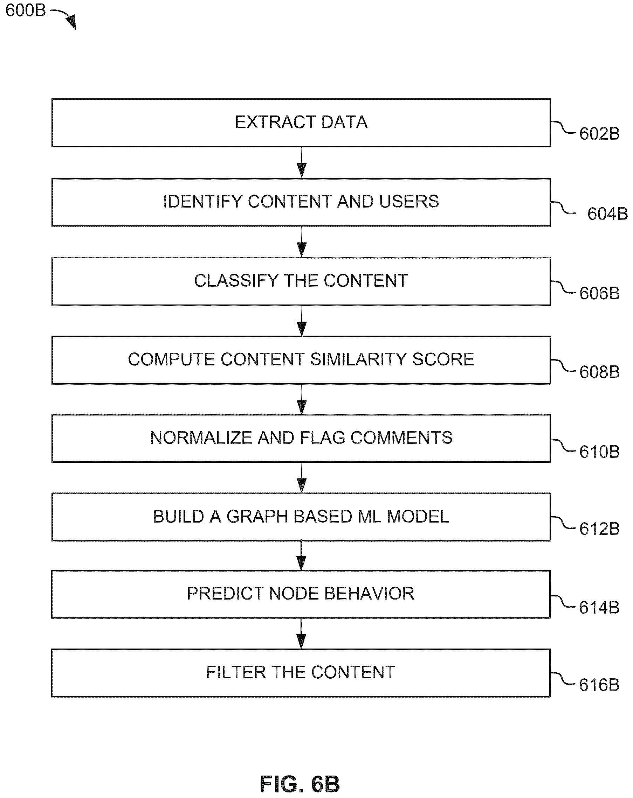

In this example implementation, during the process or step of the AE data collection, spontaneous reporting data is collected, and the AE data is extracted from web and social media contents in structured or un-structured format. For example, the AE data may include text, image, audio, and/or video in any language across the world. An example architecture illustrating a framework 500 for adverse event data collection is explained in more detail with reference to FIG. 5. The Bot detection and AI generated content is then filtered. For example, example methods 600A and 600B for detecting artificial intelligence (AI) generated content and Bot generated content are explained in more detail with reference to FIGS. 6A and 6B, respectively.

In an aspect, the process of the AE data collection involves collecting raw the AE data (structured or unstructured) from various sources, including healthcare professionals, patients or caregivers, call center representatives, clinical research associates, literature review specialist, social media, health forums, and websites, in multiple languages and formats (e.g., text, image, audio, and/or video). For example, the tools used for performing the process of AE data collection includes platform-specific APIs (e.g., social media API, website API and the like) or web scrapers for data collection, optical character recognition (OCR) for extracting text from images, speech-to-text APIs for extracting content from audio and video, LLM models with multilingual capabilities (e.g., multilingual bidirectional encoder representations from transformers (mBERT), GPT-4 and the like ) for understanding content in multiple languages, media handling APIs to extract audio tracks from video and process multimedia content, semantic scholar AI-powered search tool for academic articles, bot detection graph tool to detect automated social media accounts, supervised models, AI detection models like open AI GPT-detectors for detecting whether content has been generated by AI.

In this aspect, a supervisor agent orchestrates requests to corresponding worker agents to extract data in parallel from different sources. The supervisor agent may initialize a scanning process by determining which web and social media platforms need to be monitored based on time, data volume, or specific keywords. Further, the supervisor agent may define a search criterion, such as drug names, symptoms, side effects, or hashtags. Tasks are then prioritized, with high-severity drugs or newly released medications given higher attention. Furthermore, the supervisor agent assigns specific channels and formats to the worker agents (e.g., one worker agent may scrape a social media platform, while another worker agent handles clinical trial data extractor).

Furthermore, the worker agents may collect content in real-time or periodic intervals. For each platform, the worker agents scrape post content, user interactions (likes, shares and so on), and metadata (timestamps, user geolocation and the like). The worker agent then automatically detects the language of the content to route for appropriate language-specific processing. In addition, the worker agents adhere to platform-specific rate limits to avoid getting blocked and data is continuously streamed to system 104.

Moreover, the worker Agents may scan extracted content and apply bot detection algorithms or tools to filter out synthetic contents. Also, the worker agents apply language models designed to detect AI generated posts and discard or deprioritize content that is highly likely to be AI Generated. The worker agents flags and filters outputs and reports back to the supervisor agent. Further, the supervisor agent validates the output and only human-origin data moves forward and communicate to next a supervisor agent in queue to initialize tasks with associated group of agents and so on.

In this example implementation, during the process of the case intake and triage, AEs are documented with details like patient demographics, drug exposure, and outcomes from various data sources. Reports are prioritized based on seriousness and timeliness (e.g., serious adverse events (SAEs) require expedited reporting). The data sources include crawling and scraping data repository for raw AE data, e-mail submissions, medical records from hospitals, doctors, clinics and the like. Further, the tools used for this process are named entity recognition (NER) models to extract mentions of drugs, dosages, symptoms, and conditions from unstructured text. (e.g., drug name: "XYZ", side effects: "headache," "nausea", patient information if applicable: "female, 35 years old"). Fine-tuned LLMs on medical datasets to detect AEs in posts, personally identifiable information (PII) detection and masking using informatica, custom natural language processing (NLP) models, an ETL tool for transformation and a database connector.

In an aspect, one of worker agents (e.g., a pharmacovigilance worker agent) may validate and document case details, ensuring completeness by including essential information such as drug, dosage, timing, and patient details. Further, other workers agents prioritize incoming cases based on predefined rules (e.g., serious adverse events (hospitalization), life-threatening, or mild symptoms (nausea), unexpected reactions). Furthermore, a data entry operator worker agent organizes incoming AE data into structured formats by converting unstructured text to structured formats with fields such as drug name, symptom, dosage, severity, timestamp, and user profile data In addition, the worker agents may ensure patient privacy by anonymizing any personally identifiable information (PII) such as patient names, addresses, contact information, or medical record numbers before entering the pharmacovigilance system and populates the AE data into the database 106.

Moreover, a supervisor agent associated with the worker agents of the case intake and triage process may perform task initialization and task assignment. The supervisor agent may also ensure that triage decisions are consistent with pharmacovigilance guidelines and that cases are assigned to correct priority levels. Further, the supervisor agent escalates cases that require further investigation or clarification, such as high-risk or complex cases.

In an example implementation, a supervisor agent and worker agents may perform data validation, data deduplication, coding and causality assessment of information within safety databases and pharmacovigilance system using medical coding tools and regulatory guidelines and machine learning models for automatic assessment of relationship between the drug and AE (causality). In an aspect, a worker agent (e.g., a medical coder agent) may code adverse events, medical history, and drug names using a drug dictionary. Further, another worker agent (e.g., a case processing agent) may analyze case details, assess completeness, and ensure compliance with regulatory guidelines. Also, worker agents may enrich the data with additional metadata, such as geolocation, demographics, or time of posting for deeper analysis. Also, the worker agents may verify and enriches the structured dataset by cross-referencing with clinical trial data, prior AE reports, and regulatory databases. Moreover, the worker agents may de-duplicate posts with similar content using fuzzy string matching or clustering algorithms (e.g., Levenshtein distance (also known and referenced as edit distance), k-means clustering and the like). Further, another worker agent (e.g., a pharmacovigilance physician agent) may perform causality assessments to determine whether the adverse event is likely related to the drug using algorithms (e.g., Naranjo Scale and the like). Furthermore, the supervisor agent may perform a secondary review of the case, ensuring that the adverse event is accurately coded and that the narrative is clear and complete. The supervisor agent may also review the causality assessment to confirm the correct decision.

In an example implementation, during the step of signal detection and analysis, new or rare safety concerns are identified using statistical methods (e.g., relevance scoring, severity filtering and the like) and data mining from large safety databases. Also, significance of the signals and their potential impact on patient safety is analyzed. In an aspect, a supervisor agent may define formatting standards and necessary data transformations for regulatory compliance. Further, a worker agent (e.g., a data selector worker) may filter relevant ADEs based on severity, novelty, or frequency and store fully structured AE records in a regulatory-compliant format. Furthermore, the worker agents may mine and analyze large safety databases for potential signals. The worker agents may use statistical tools to identify new or unexpected safety trends (e.g., using an empirical signal or SAS). In addition, the worker agents may aggregate data from different sources (clinical trials, spontaneous reports, etc.) and run statistical models to assess any correlation between drug use and adverse events. Moreover, the supervisor agent may assess results of signal detection and evaluates the significance and relevance of identified signals. The supervisor agent then decides whether to initiate further investigation based on the strength and relevance of the signal. The supervisor agent may recommend more in-depth analysis, such as additional data mining or expert medical review. For example, data used for signal detection and analysis include pharmacovigilance databases, scientific literature, previous clinical trial data, and global AE databases and tools may include statistical analysis software for data mining and signal detection (e.g., Bayesian analysis, disproportionality analysis), machine learning algorithms for advanced signal detection and data mining and data visualization tools for visualizing data trends.

In an example implementation, during the step of risk management, risk minimization measures like label changes, warnings, restricted use, or patient education programs are implemented. Further, a risk management plan (RMP) is developed for high-risk drugs to outline actions to monitor and mitigate risks. In an aspect, a worker agent (e.g., a risk management specialist agent) may develop specific risk management strategies based on the identified risks, such as changes to labeling, restrictions on use, or patient monitoring programs. The worker agent may also create and update the RMP for high-risk drugs, including post-marketing surveillance activities. Furthermore, a supervisor agent (e.g., a regulatory affair specialist agent) may review and approve proposed risk mitigation plans, ensuring that they align with regulatory requirements and industry best practices. The supervisor agent may also ensure that all stakeholders (regulatory authorities, healthcare professionals) are appropriately informed of new risk management measures, and they approve communications to external parties. For example, data used for performing this step includes historical risk data, case studies, clinical trial outcomes, expert opinions, and regulatory requirements and tools used by the agents include risk management software, AI models that predict potential risks based on past data and regulatory tools for tracking RMP submissions.

In an example implementation, during the step of regulatory reporting, the structured AE data is compiled into regulatory documents for reporting to health authorities as expedited reports or periodic reports. In an aspect, a worker agent (e.g., regulatory affair specialist agent) may prepare individual case safety reports (ICSRs) for serious adverse events (SAEs) within the required timelines (e.g., 15-day expedited reporting), periodic safety update reports (PSURs) and periodic benefit-risk evaluation reports (PBRERs). Further, a supervisor agent (e.g., compliance officer agent) may ensure that regulatory reports are complete, comply with global pharmacovigilance standards, and meet submission deadlines. The supervisor agent also signs off on a final version of reports before they are submitted to regulatory authorities. For example, information used for performing this step includes regulatory guidelines and templates and case processing data.

In an example implementation, during the process of continuous benefit-risk evaluation, benefit-risk balance is monitored throughout the product's lifecycle to ensure that the drug's benefits outweigh its risks with updated scientific data. For example, data used for this process include post-marketing data, clinical trial data, new literature, AE reports and patient feedback. Tools used by the agents include tools for modeling and simulating the benefit-risk profile (e.g., ClinRisk) and data analytics Tools for continuous data analysis. In an aspect, a safety monitoring worker agent compiles data on the safety and efficacy of a drug and assess its overall benefit-risk balance based on latest information. Further, a scientific advisory supervisor agent, often in collaboration with senior medical experts, may review benefit-risk analysis and provide recommendations for any changes to a product label, usage instructions, or risk minimization strategies. Also, the scientific advisory supervisor agent may ensure continuous monitoring and periodic reassessment of the benefit-risk balance or profile as new safety data emerges.

In an example implementation, during the step of communication of safety information, healthcare professionals, patients, and regulatory bodies are informed about newly identified risks or safety updates. Also, dissemination of updated product labeling, safety notices, and educational materials is performed. For example, data used in this step include AE reports, regulatory updates, scientific literature and tools used include communication platforms and regulatory communication tools. In an aspect, a medical affair worker agent may create communication materials (e.g., safety alerts) to educate healthcare providers on updated safety information. Further, a public relations specialist agent may create communication material (e.g., updates to product labels, safety notices and recalls to the public). Furthermore, a regulatory communication supervisor agent may review and approve safety communications before dissemination. The agents may ensure that the communication aligns with company policies, regulatory guidelines, and is factually accurate.

In an example implementation, during the step of audit and compliance, adherence to good pharmacovigilance practices (GVP) and regulatory guidelines is ensured and regular audits and inspections are conducted by regulatory authorities. In an aspect, a quality assurance auditor worker agent prepares for audits and ensures that pharmacovigilance records, data, and reports are well-documented and easily accessible for auditing purposes. Further, another worker agent may inspect pharmacovigilance systems for compliance GVPs. Also, a supervisor agent leads the auditing process, ensuring that pharmacovigilance activities are fully compliant with GVPs. The supervisor agent may review audit reports and take corrective actions when necessary and ensure that any corrective actions resulting from audits are implemented and tracked for effectiveness.

Referring now to FIG. 4 that depicts an example method 400 illustrating interaction between supervisor agents and corresponding worker agents while performing various tasks, in accordance with implementations of the present disclosure. In an example implementation, the method 400 depicts interaction between a group of agents including a supervisor agent1 and corresponding worker agents. In the method 400, the supervisor agent1 performs task allocation, performance evaluation, communication with worker agents, decision-making, optimization and dynamic task reallocation 402 to the corresponding worker agents. Further, the method 400 may include executing a task by one or more of the worker agents and sending feedback 404 to the supervisor agent1. Furthermore, the method 400 may include determining whether the task is understood by the one or more worker agents 406. Then, the method 400 may include executing the task 408 by the one or more worker agents when the task is understood. The method 400 may include requesting clarification by the supervisor agent1 when the task is not understood. Upon executing the task, the method 400 includes determining whether the task is executed without any errors 410. The method 400 may include sending task completion notification to the supervisor agent1 by the one or more worker agents when the task is executed without any errors. The method 400 may then include performing error handling 412 when the task is executed with one or more errors.

Moreover, the method 400 includes determining whether the one or more errors are recoverable 414. When the one or more errors are recoverable, the method 400 includes retrying and adjusting the task 416 based on clarification or resolution instructions sent by the supervisor agent1. The method 400 may include escalating to the supervisor agent1 when the one or more errors are not recoverable. Also, the method 400 may include determining whether retries are exhausted 418. The method 400 may then include executing the task and notifying the supervisor agent 420 when the retries are not exhausted. Further, the method 400 may include escalating to the supervisor agent1 when the retries are exhausted. In an aspect, the supervisor agent1 may re-allocate or reassign the task to a new or different worker agent for executing the task. In some examples, an output of the group of agents is communicated to a different group of agents 422, where the various tasks are executed as described above using the output.

FIG. 5 depicts an example architecture illustrating a framework 500 for the AE data collection, in accordance with implementations of the present disclosure. As shown in FIG. 5, the framework 500 includes a supervisor agent 502, a plurality of worker agents 504A-F, a data source (e.g., the data sources 102A-N) and tools 506 for the group of worker agents 504A, a data source and tools 516 for the group of worker agents 504B, respectively, and data sources and tools 518A-D for the worker agents 504C-504F, respectively, a backstory and task optimizer 508 (e.g., the backstory and task optimizer 210), a message queue 510, a data storage repository 512 and internal and external databases 514 (i.e., the database 226 and model database 222). For example, each of the supervisor agent 502 and the worker agents 504A-F include a high-performance server with a graphics processing unit (GPU) or tensor processing unit (TPU) for fast processing. In this example, the worker agents 504A-C may be cloud-based LLMs, and the worker agents 504D-F may be on-premsis LLM. In an aspect, data available in the data sources 506 and data sources 518A-D can be one or more of structured data, unstructured data, multi-modal data (e.g., text, video, image, and audio) and the like in any language. Also, the data source and tools 506, the data source and tools 516, the data source and tools 518A-D can recite in a web and social media scraper server (not shown in FIG. 5). The web and social media scraper server is a multi-core processor that can handle parallel scraping tasks with scraping tools to perform data extraction and collection and filter out the non-human content, ensuring authentic user generated data.

In an example implementation, the supervisor agent 502 continuously communicates with the worker agents 504A-F via the message queue 510. The supervisor agent 502 may perform task initialization to start a scanning process across various platforms and detect Bot and AI generated content. Further, the supervisor agent 502 may distribute tasks to the worker agents 504A-F efficiently. Also, the supervisor agent 502 optimize backstories and oversees the worker agents 504A-F via the backstory and task optimizer 508. For example, information associated with the agents 502 and 504A-504F, the tasks performed by the agents 502 and 504A-504F, tools, the backstories and the like are stored in the data storage repository 512 and the internal and external databases 514.

Furthermore, in this example implementation, the worker agents 504A extracts social media data using the data source and tools 506. For example, the data source 506 include web and social media channels, health and patient forums, medical research, blogs and the like. The tools 506 may include crawling and scraping tools. In addition, the worker agents 504B detects Bot and AI generated content using the data source and tools 516. For example, the tools 516 may include Bot detection graph tool and AI detection tool. Moreover, the worker agent 504C collects literature and research data using the data source and tools 518A. For example, the data source and tools 518A includes medical journals and AI-powered research tools. Also, the worker agent 504D collects clinical research data using the data source and tools 518B. For example, the data source and tools 518B includes clinical trial data and extract, transform and load (ETL) tools with database connectors.

Further, the worker agent 504E collects call center data using the data source and tools 518C. For example, the data source and tools 518C includes call center data and ETL tools with database connectors. Furthermore, the worker agent 504F collects information regarding interaction between health care professional (HCP) and patient using the data source and tools 518D. For example, the data source and tools 518D includes spontaneous reports database and tools, such as VigiBase, FDA's FAERS, medicine and healthcare regulatory agency (MHRA) yellow Card. In some examples, the worker agents 504A-F may communicate results of each of the worker agents 540A-F to the supervisor agent 502.

FIG. 6A is a flow diagram that represents an example method 600A for detecting AI generated content using GenDectGPT, in accordance with implementations of the present disclosure. In an example implementation, the method 600A may include data cleaning and preprocessing 602A. The textual data may be cleaned and pre-processed to remove noise, inconsistencies, and irrelevant information, thereby improving model's performance. Further, the method 600A may include text encoding with embeddings from language models (ELMO) tokenizer 604A. For example, the pre-processed text is encoded using the ELMO tokenizer, transforming it into a numerical representation suitable for input into a detection model. Furthermore, the method 600A may include creating a label dataset 606A by clustering similar embeddings using active learning to tag the AI generated content. Also, similarities with known model datasets are found and historical pattern with the text is detected.

In addition, the method 600A may perform model training 608A where the GenDectGPT, or a small language model, which is a sequence classification model is trained on a labelled dataset to learn distinctive characteristics of genuine and AI-generated text. Moreover, the method 600A may detect or predict new content or text 610A. The trained model may be used to predict whether the new, unseen text is likely to be AI generated content. Also, the method 600A may include analysing predictions or results 612A. The predictions, along with associated confidence scores, may be saved in a file of the database 226 for review and analysis.

In an example, there are social media posts discussing various drugs, side effects, or symptoms, with terms like slang, emojis, and misspellings. First, irrelevant symbols, hashtags, or usernames (e.g., @user123) are removed, common spelling errors are corrected, and stop words (e.g., "the," "is," "at") are eliminated. Further, text is converted to lowercase (or uppercase) for uniformity and sentences are tokenized to standardize structure. Each cleaned post is then encoded using the ELMO tokenizer. For instance, the post "This drug made me feel dizzy" is tokenized and converted into numerical embeddings using ELMO. These embeddings capture the contextual meaning of words like "dizzy" based on surrounding text, which may improve downstream analysis.

Furthermore, embeddings from similar posts are grouped, such as those discussing dizziness or nausea, into clusters. Active learning can be used to tag ambiguous posts as "potential side effect" or "not drug-related" by having a human-in-the-loop verify and adjust tags, thus refining the AI's understanding. Also, patterns similar to previously identified side effects from known medical datasets are found and clusters that align with historical data trends are detected.

In addition, a sequence classification model, GenDectGPT, is trained on the labelled dataset, distinguishing between genuine user experiences and potential AI-generated or promotional content. The model learns linguistic patterns indicative of real user-generated content versus generated content, fine-tuning on the nuances in social media discussions around drugs.

With the trained model, input a new post that claims, "This drug had no side effects, and it's a miracle cure!." The language patterns and context are analysed using the model to predict if the post is genuine or potentially AI generated, based on training from labelled examples. For every prediction, model's output, such as the confidence score, is stored in a comma-separated values (CSV) file of the database 226. The file may help with further analysis of detected trends, allowing medical data analysts to assess the prevalence of real versus generated content about specific drugs.

FIG. 6B is a flow diagram representing an example method 600B for detecting Bot generated content, respectively, in accordance with implementations of the present disclosure. In an example implementation, the method 600B may include extracting data 602B from social media platforms including connection details, directions and comments. Further, the method 600B may include initial identification 604B of users based on the extracted data. The method 600B may then include analyzing the content for possible bot behaviour. In addition, the method 600B may include classifying comments and/or content 606B using the GenDectGPT for further analysis. Moreover, the method 600B may include generating a similarity score for new comments 608B to detect patterns of the bot or repeated content. Also, the method 600B may include normalizing and flagging the comments 610B by processing each comment and associating the comments with users. The comments are then flagged as either 1 - True (unique) or 0 - Repeated/Fraud.

Further, the method 600B may include building a graph-based machine learning model 612B to classify nodes and focus on identifying users. Furthermore, the method 600B may include predicting behaviour of nodes to further refine user identification 614B. If a user's reality score is low, then the method 600B may include filtering out associated content 616B.

For example, data is collected from a social media feed, such as connection details including followers, likes, retweets and the like, directions including relationship and interaction patterns between users and comments including specific comments or replies to tweets. Further, user profiles are analyzed to detect bot-like patterns. In an example, users posting at high frequency 24/7, with minimal engagement other than reposting. Furthermore, comment patterns are reviewed to spot inauthentic or spammy text. In an example, a comment repeatedly promoting a website across different user posts.

Moreover, a comment like "Check out this amazing deal" is classified as likely spam content using GenDectGPT. The GenDectGPT may help distinguish between genuine engagement and potentially automated or promotional content. Also, a similarity score is calculated for a comment, such as "Follow this link for prizes." If this message has been used 100+ times in the network, a high similarity score indicates repetitive content and suggests it may be spam. Further, a comment may be flagged as "1 - True" if it's unique or "0 - repeated or fraud" if it closely matches other comments tagged as suspicious or spam. Furthermore, a graph model is built where each node represents a user and edges represent connections (e.g., follows, likes). The model then classifies nodes with low engagement diversity and high repetition as possible bots. Behavior is then predicted based on previous actions. If a user mostly reposts others' content with no unique input, the model might classify the user as a potential bot due to lack of organic activity. If a user's reality score falls below a threshold (indicating low credibility), the content is then automatically filtered, so the posts no longer appear in broader feeds.

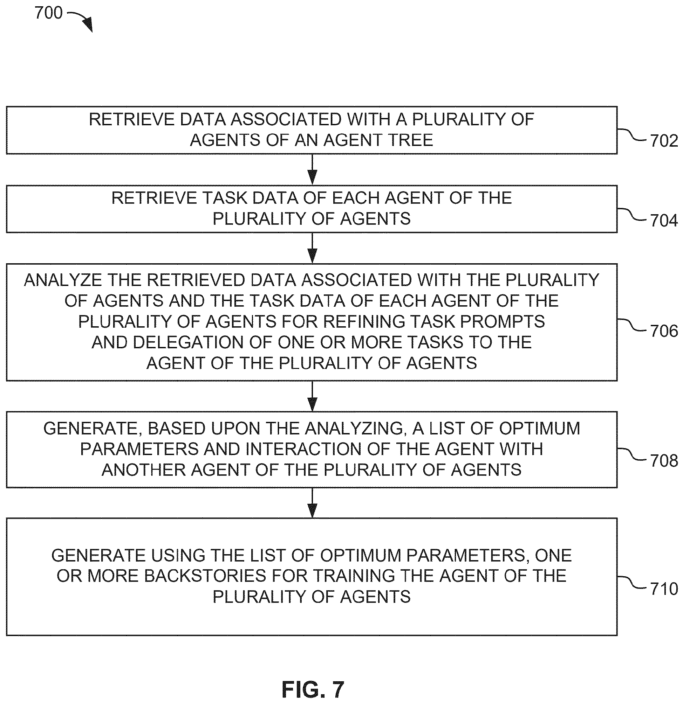

FIG. 7 is a flow diagram that represents an example computer implemented method 700 for automatic reporting of an ADR, in accordance with implementations of the present disclosure. In some implementations, the method 700 may be executed by the processor 202 (including the one or more processors), as described in relation to FIGS 1-6B.

In an example implementation, the method 700 may include retrieving data 702 associated with a plurality of agents of an agent tree. The plurality of agents may include one or more supervisor agents, and one or more working agents associated with each supervisor agent of the one or more supervisor agents. For example, the data associated with the plurality of agents may include persona data of an agent, purpose and alignment of the agent, a large language model (LLM) and corresponding configuration data of the large language model, a list of one or more tools or applications and corresponding application programing interface integrated with the agent, a list of document functions or application programing interfaces invoked by the large language model, and/or one or more operational parameters. In this example, the list of one or more tools integrated with the agent comprises one or more of a retrieval augmented generation (RAG) pipeline, a Genetic Detection Generative Pre-Trained Transformer (GenDectGPT), a Simulated Interactive Model using GPT (SIMGPT), a Bot Detector, internal database connectors, external database connectors, and/or storage connectors.

Further, the method 700 may include retrieving task data 704 of each agent of the plurality of agents. For example, the task data may include description of a task assigned to the agent, a goal or deliverable associated with the task assigned to the agent, a list of one or more functions to be called upon completion of the task assigned to the agent, and/or a flag identifying whether the task assigned to the agent supports asynchronous operations.

Furthermore, the method 700 may include analyzing 706 the retrieved data associated with the plurality of agents and the task data of each agent of the plurality of agents for refining task prompts and delegation of one or more tasks to an agent of the plurality of agents. In an example implementation, to analyze the retrieved data associated with the plurality of agents and the task data of each agent of the plurality of agents, the method 700 may include identifying a communication type of communication of the agent with the other agent. The communication type may be a sequential communication, a hierarchical communication, and/or a consensual communication. Further, the method 700 may include delegating, based upon the identified communication type, a data collection task for generating the one or more backstories to the agent of the plurality of agents. The data collection task may include optimizing a prompt, extracting data using the optimized prompt, and/or cleaning the extracted data.

In this example implementation, the method 700 may further include generating an agent-level log, a task-level log, and/or a communication log. Furthermore, the method 700 may include identifying one or more of a bottleneck in agent communication, an opportunity for retry optimization, a prompt refinement, and/or enhanced delegation-and-alignment strategies of an agent selection.

Based upon the analyzing, the method 700 may include generating 708 a list of optimum parameters and interaction of the agent with other agent of the plurality of agents. In an example implementation, to generate the list of optimum parameters and interaction of the agent with other agent of the plurality of agents, the method 700 may include generating a plurality of node features, a plurality of edge features, and/or a target variable. For example, the plurality of node features corresponds with features of the agent and the agent represent a node of a network graph. The plurality of edge features may correspond to vector representation of parameters including a maximum number of iterations, a maximum number of requests per minute, a maximum number of retry limits, a memory, a cache, and/or a step callback.

Further, in this example implementation, the method 700 may include splitting data of each edge of the network graph into one or more of a training dataset, a validation dataset, and/or a test dataset. Also, the method 700 may include capturing a local graph structure and a unified representation of each edge of the network graph by aggregating the plurality of node features and the plurality of edge features.

Using the list of optimum parameters, the method 700 may include generating one or more backstories 710 for training the agent of the plurality of agents. For example, the agent is trained for automatic reporting of the ADR.

In some examples, the method 700 may include retrieving adverse drug reaction data from one or more of a social media source, a clinical trial, a medical journal, a data repository storing data of a similar drug, and a scientific study. Further, the method 700 may include re-analyzing the adverse drug reaction data along with the retrieved data associated with the plurality of agents and the task data of each agent of the plurality of agents for refining the task prompts and delegation of the one or more tasks to the agent of the plurality of agents.

In some examples, the method 700 may include training a machine learning model for training the agent of the plurality of agents by applying a regression loss function. Further, the method 700 includes validating the machine learning model based upon a prediction of the trained machine learning model.

Implementations of the present disclosure provide technical solutions to multiple technical problems that arise in the context of reporting ADRs. The proposed methodology establishes communication between different agents as security is required between on-prem and SaaS LLM-based agents. Also, the implementations of the present disclosure uses multiple AI based agent tools to identify bots using bot detection graph and detect AI generated content. Therefore, the reporting of the ADRs is performed using filtered data with improved accuracy, confidence level, rate of true positives, and/or the like. This, in turn, may improve response time, conserve computing resources, networking resources, and/or the like that otherwise have been overutilized in analyzing unnecessarily addressing false positive, false negatives, and/or the like.

Implementations of the present disclosure optimizes description, back stories and task prompts of the agents as per the output and feedback. Therefore, the proposed methodology improves overall process of reporting of the ADRs.

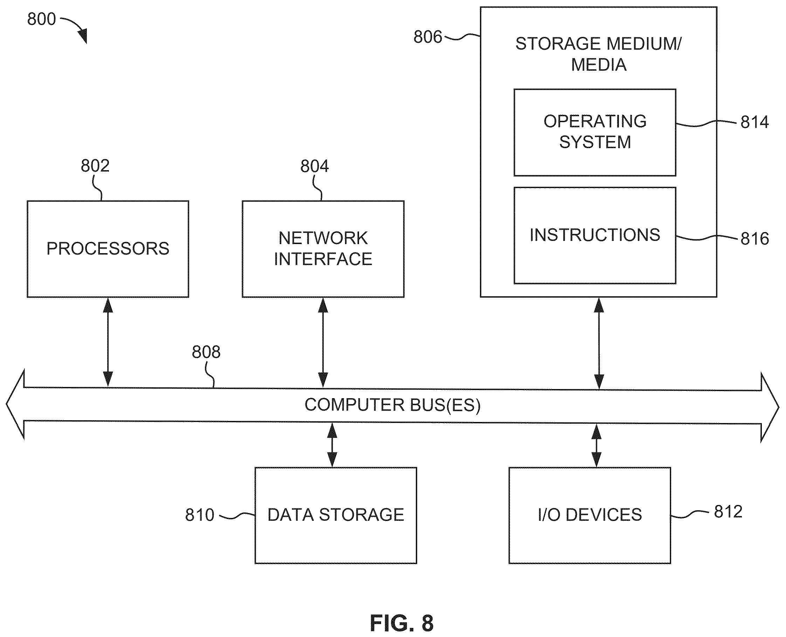

FIG. 8 illustrates a computer system 800 (i.e., the ADR reporting system 104) that may be used to implement the method of automatically reporting ADRs, in accordance with implementations of the present disclosure. More particularly, computing machines such as desktops, laptops, smartphones, tablets, and wearables which may be used to perform the software testing. The computer system 800 may include additional components not shown and that some of the process components described may be removed and/or modified. In another example, a computer system 800 may be deployed on external-cloud platforms such as cloud, internal corporate cloud computing clusters, organizational computing resources, and/or the like.

The computer system 800 includes processor(s) 802, such as a central processing unit, ASIC or another type of processing circuit, input/output devices 804, such as a display, mouse keyboard, etc., a network interface 806, such as a Local Area Network (LAN), a wireless 802.11x LAN, a 3G or 4G mobile WAN or a WiMax WAN, and a computer-readable medium 808. Each of these components may be operatively coupled to a bus 810. The computer-readable medium 808 may be any suitable medium that participates in providing instructions to the processor(s) 802 for execution. For example, the computer-readable medium 808 may be non-transitory or non-volatile medium, such as a magnetic disk or solid-state non-volatile memory or volatile medium such as RAM. The instructions or modules stored on the computer-readable medium 808 may include machine-readable instructions 812 executed by the processor(s) 802 that cause the processor(s) 802 to perform the methods and functions of the system 104.

The system 104 may be implemented as software stored on a non-transitory processor-readable medium and executed by the processor(s) 802. For example, the computer-readable medium 808 may store an operating system 814, such as MAC OS, MS WINDOWS, UNIX, or LINUX, and code, for the system 104. The operating system 814 may be multi-user, multiprocessing, multitasking, multithreading, real-time, and the like. For example, during runtime, the operating system 814 is running and the code for the computer system 800 is executed by the processor(s) 802.

The computer system 800 may include a data storage 816, which may include non-volatile data storage. The data storage 816 stores any data used or generated by the system 104.

The network interface 806 connects the computer system 800 to internal systems for example, via a LAN. Also, the network interface 806 may connect the computer system 800 to the Internet. For example, the computer system 800 may connect to web browsers and other external applications and systems via the network interface 806.

What has been described and illustrated herein is an example along with some of its variations. The terms, descriptions, and figures used herein are set forth by way of illustration only and are not meant as limitations. Many variations are possible within the spirit and scope of the subject matter, which is intended to be defined by the following claims and their equivalents.

Implementations and all of the functional operations described in this specification may be realized in digital electronic circuitry, or in computer software, firmware, or hardware, including the structures disclosed in this specification and their structural equivalents, or in combinations of one or more of them. Implementations may be realized as one or more computer program products (i.e., one or more modules of computer program instructions encoded on a computer readable medium for execution by, or to control the operation of, data processing apparatus). The computer readable medium may be a machine-readable storage device, a machine-readable storage substrate, a memory device, a composition of matter effecting a machine-readable propagated signal, or a combination of one or more of them. The term "computing system" encompasses all apparatus, devices, and machines for processing data, including by way of example a programmable processor, a computer, or multiple processors or computers. The apparatus may include, in addition to hardware, code that creates an execution environment for the computer program in question (e.g., code that constitutes processor firmware, a protocol stack, a database management system, an operating system, or any appropriate combination of one or more thereof). A propagated signal is an artificially generated signal (e.g., a machine-generated electrical, optical, or electromagnetic signal) that is generated to encode information for transmission to suitable receiver apparatus.

A computer program (also known as a program, software, software application, script, or code) may be written in any appropriate form of programming language, including compiled or interpreted languages, and it may be deployed in any appropriate form, including as a stand-alone program or as a module, component, subroutine, or other unit suitable for use in a computing environment. A computer program does not necessarily correspond to a file in a file system. A program may be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program may be deployed to be executed on one computer or on multiple computers that are located at one site or distributed across multiple sites and interconnected by a communication network.

The processes and logic flows described in this specification may be performed by one or more programmable processors executing one or more computer programs to perform functions by operating on input data and generating output. The processes and logic flows may also be performed by, and apparatus may also be implemented as, special purpose logic circuitry (e.g., an FPGA (field programmable gate array) or an ASIC (application specific integrated circuit)).

Processors suitable for the execution of a computer program include, by way of example, both general and special purpose microprocessors, and any one or more processors of any appropriate kind of digital computer. Generally, a processor will receive instructions and data from a read only memory or a random-access memory or both. Elements of a computer may include a processor for performing instructions and one or more memory devices for storing instructions and data. Generally, a computer includes or is operatively coupled to receive data from or transfer data to, or both, one or more mass storage devices for storing data (e.g., magnetic, magneto optical disks, or optical disks). However, a computer need not have such devices. Moreover, a computer may be embedded in another device (e.g., a mobile telephone, a personal digital assistant (PDA), a mobile audio player, a Global Positioning System (GPS) receiver). Computer readable media suitable for storing computer program instructions and data include all forms of non-volatile memory, media and memory devices, including by way of example semiconductor memory devices (e.g., EPROM, EEPROM, and flash memory devices); magnetic disks (e.g., internal hard disks or removable disks); magneto optical disks; and CD ROM and DVD-ROM disks. The processor(s) 802 and the memory may be supplemented by, or incorporated in, special purpose logic circuitry.

To provide for interaction with a user, implementations may be realized on a computer having a display device (e.g., a CRT (cathode ray tube), LCD (liquid crystal display) monitor) for displaying information to the user and a keyboard and a pointing device (e.g., a mouse, a trackball, a touch-pad), by which the user may provide input to the computer. Other kinds of devices may be used to provide for interaction with a user as well; for example, feedback provided to the user may be any appropriate form of sensory feedback (e.g., visual feedback, auditory feedback, tactile feedback); and input from the user may be received in any appropriate form, including acoustic, speech, or tactile input.

Implementations may be realized in a computing system that includes a back end component (e.g., as a data server), a middleware component (e.g., an application server), and/or a front end component (e.g., a client computer having a graphical user interface or a Web browser, through which a user may interact with an implementation), or any appropriate combination of one or more such back end, middleware, or front end components. The components of the system may be interconnected by any appropriate form or medium of digital data communication (e.g., a communication network). Examples of communication networks include a local area network ("LAN") and a wide area network ("WAN"), e.g., the Internet. The computing system may include clients and servers. A client and server are generally remote from each other and interact through a communication network. The relationship of client and server arises by virtue of computer programs running on the respective computers and having a client-server relationship to each other.

While this specification contains many specifics, these should not be construed as limitations on the scope of the disclosure or of what may be claimed, but rather as descriptions of features specific to particular implementations. Certain features that are described in this specification in the context of separate implementations may also be implemented in combination in a single implementation. Conversely, various features that are described in the context of a single implementation may also be implemented in multiple implementations separately or in any suitable sub-combination. Moreover, although features may be described above as acting in certain combinations and even initially claimed as such, one or more features from a claimed combination may in some cases be excised from the combination, and the claimed combination may be directed to a sub-combination or variation of a sub-combination. Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the implementations described above should not be understood as requiring such separation in all implementations, and it should be understood that the described program components and systems may generally be integrated together in a single software product or packaged into multiple software products.

A number of implementations have been described. Nevertheless, it will be understood that various modifications may be made without departing from the spirit and scope of the disclosure. For example, various forms of the flows shown above may be used, with steps reordered, added, or removed. Accordingly, other implementations are within the scope of the following claims.

## Claims

1. A computer-implemented method for automatic reporting of an adverse drug reaction, the computer-implemented method comprising:
retrieving, by one or more processors of one or more computing devices, data associated with a plurality of agents of an agent tree;
retrieving, by the one or more processors of the one or more computing devices, task data of each agent of the plurality of agents;
analyzing, by the one or more processors of the one or more computing devices, the retrieved data associated with the plurality of agents and the task data of each agent of the plurality of agents for refining task prompts and delegation of one or more tasks to an agent of the plurality of agents;
generating, by the one or more processors of the one or more computing devices, based upon the analyzing, a list of optimum parameters and interaction of the agent with other agent of the plurality of agents; and
generating, by the one or more processors of the one or more computing devices, using the list of optimum parameters, one or more backstories for training the agent of the plurality of agents, wherein the agent is trained for automatic reporting of the adverse drug reaction.

2. The computer-implemented method of claim 1, wherein the data associated with the plurality of agents includes: persona data of an agent, purpose and alignment of the agent, a large language model and corresponding configuration data of the large language model, a list of one or more tools or applications and corresponding application programing interface integrated with the agent, a list of document functions or application programing interfaces invoked by the large language model, and/or one or more operational parameters.

3. The computer-implemented method of claim 2, wherein the list of one or more tools integrated with the agent comprises one or more of: a retrieval augmented generation (RAG) pipeline, a Genetic Detection Generative Pre-Trained Transformer (GenDectGPT), a Simulated Interactive Model using GPT (SIMGPT), a Bot Detector, internal database connectors, external database connectors, and/or storage connectors.

4. The computer-implemented method of any one of claims 1 to 3, wherein the task data includes description of a task assigned to the agent, a goal or deliverable associated with the task assigned to the agent, a list of one or more functions to be called upon completion of the task assigned to the agent, and/or a flag identifying whether the task assigned to the agent supports asynchronous operations.

5. The computer-implemented method of any one of claims 1 to 4, further comprising:
retrieving, by the one or more processors of the one or more computing devices, adverse drug reaction data from one or more of: a social media source, a clinical trial, a medical journal, a data repository storing data of a similar drug, a scientific study; and
re-analyzing, by the one or more processors of the one or more computing devices, the adverse drug reaction data along with the retrieved data associated with the plurality of agents and the task data of each agent of the plurality of agents for refining the task prompts and delegation of the one or more tasks to the agent of the plurality of agents.

6. The computer-implemented method of any one of claims 1 to 5, wherein the plurality of agents includes one or more supervisor agents, and one or more working agents associated with each supervisor agent of the one or more supervisor agents.

7. The computer-implemented method of any one of claims 1 to 6, wherein analyzing the retrieved data associated with the plurality of agents and the task data of each agent of the plurality of agents comprises:
identifying, by the one or more processors of the one or more computing devices, a communication type of communication of the agent with the other agent, wherein the communication type is a sequential communication, a hierarchical communication, and/or a consensual communication; and
delegating, by the one or more processors of the one or more computing devices, based upon the identified communication type, a data collection task for generating the one or more backstories to the agent of the plurality of agents, wherein the data collection task includes optimizing a prompt, extracting data using the optimized prompt, and/or cleaning the extracted data; and preferably wherein analyzing the retrieved data associated with the plurality of agents and the task data of each agent of the plurality of agents further comprises:
generating, by the one or more processors of the one or more computing devices, an agent-level log, a task-level log, and/or a communication log; and
identifying, by the one or more processors of the one or more computing devices, one or more of: a bottleneck in agent communication, an opportunity for retry optimization, a prompt refinement, and/or enhanced delegation-and-alignment strategies of an agent selection.

8. The computer-implemented method of any one of claims 1 to 7, wherein generating the list of optimum parameters and interaction of the agent with the other agent of the plurality of agents comprises:
generating, by the one or more processors of the one or more computing devices, a plurality of node features, a plurality of edge features, and/or a target variable, wherein the plurality of node features corresponds with features of the agent and the agent represent a node of a network graph, wherein the plurality of edge features corresponds vector representation of parameters including a maximum number of iterations, a maximum number of requests per minute, a maximum number of retry limits, a memory, a cache, and/or a step callback;
splitting, by the one or more processors of the one or more computing devices, data of each edge of the network graph into at least a training dataset, a validation dataset, and/or a test dataset; and
capturing, by the one or more processors of the one or more computing devices, a local graph structure and a unified representation of each edge of the network graph by aggregating the plurality of node features and the plurality of edge features; and the method optionally further comprising:
training, by the one or more processors of the one or more computing devices, a machine learning model for training the agent of the plurality of agents by applying a regression loss function; and
validating, by the one or more processors of the one or more computing devices, the machine learning model based upon a prediction of the trained machine learning model.

9. A system for automatic reporting of an adverse drug reaction comprising:
at least one memory configured to store machine-executable instructions; and
at least one processor communicatively coupled with the at least one memory, and configured to execute the machine-executable instructions to perform operations comprising:
retrieving data associated with a plurality of agents of an agent tree;
retrieving task data of each agent of the plurality of agents;
analyzing the retrieved data associated with the plurality of agents and the task data of each agent of the plurality of agents for refining task prompts and delegation of one or more tasks to an agent of the plurality of agents;
generating based upon the analyzing, a list of optimum parameters and interaction of the agent with other agent of the plurality of agents; and
generating using the list of optimum parameters, one or more backstories for training the agent of the plurality of agents, wherein the agent is trained for automatic reporting of the adverse drug reaction.

10. The system of claim 9, wherein the data associated with the plurality of agents includes:
persona data of an agent, purpose and alignment of the agent, a large language model and corresponding configuration data of the large language model, a list of one or more tools or applications and corresponding application programing interface integrated with the agent, a list of document functions or application programing interfaces invoked by the large language model, and/or one or more operational parameters, wherein the list of one or more tools integrated with the agent comprises one or more of: a retrieval augmented generation (RAG) pipeline, a Genetic Detection Generative Pre-Trained Transformer (GenDectGPT), a Simulated Interactive Model using GPT (SIMGPT), a Bot Detector, internal database connectors, external database connectors, and/or storage connectors; and/or
wherein the task data includes description of a task assigned to the agent, a goal or deliverable associated with the task assigned to the agent, a list of one or more functions to be called upon completion of the task assigned to the agent, and/or a flag identifying whether the task assigned to the agent supports asynchronous operations.

11. The system of claim 9 or 10, wherein the operations further comprise:
retrieving adverse drug reaction data from one or more of: a social media source, a clinical trial, a medical journal, a data repository storing data of a similar drug, a scientific study; and
re-analyzing the adverse drug reaction data along with the retrieved data associated with the plurality of agents and the task data of each agent of the plurality of agents for refining the task prompts and delegation of the one or more tasks to the agent of the plurality of agents;
and/or
wherein the plurality of agents includes one or more supervisor agents, and one or more working agents associated with each supervisor agent of the one or more supervisor agents.

12. The system of any one of claims 9 to 11, wherein analyzing the retrieved data associated with the plurality of agents and the task data of each agent of the plurality of agents comprises:
identifying a communication type of communication of the agent with the other agent, wherein the communication type is a sequential communication, a hierarchical communication, and/or a consensual communication; and
delegating based upon the identified communication type, a data collection task for generating the one or more backstories to the agent of the plurality of agents, wherein the data collection task includes optimizing a prompt, extracting data using the optimized prompt, and/or cleaning the extracted data;
wherein analyzing the retrieved data associated with the plurality of agents and the task data of each agent of the plurality of agents optionally further comprises:
generating an agent-level log, a task-level log, and/or a communication log; and
identifying one or more of: a bottleneck in agent communication, an opportunity for retry optimization, a prompt refinement, and/or enhanced delegation-and-alignment strategies of an agent selection.

13. The system of any one of claims 9 to 12, wherein generating the list of optimum parameters and interaction of the agent with the other agent of the plurality of agents comprises:
generating a plurality of node features, a plurality of edge features, and/or a target variable, wherein the plurality of node features corresponds with features of the agent and the agent represent a node of a network graph, wherein the plurality of edge features corresponds vector representation of parameters including a maximum number of iterations, a maximum number of requests per minute, a maximum number of retry limits, a memory, a cache, and/or a step callback;
splitting data of each edge of the network graph into at least a training dataset, a validation dataset, and/or a test dataset; and
capturing a local graph structure and a unified representation of each edge of the network graph by aggregating the plurality of node features and the plurality of edge features.

14. The system of claim 13, wherein the operations further comprise:
training a machine learning model for training the agent of the plurality of agents by applying a regression loss function; and
validating the machine learning model based upon a prediction of the trained machine learning model.

15. At least one non-transitory computer-readable media comprising machine-executable instructions stored thereon, which, when executed by at least one processor of at least one computing device configured for automatic reporting of an adverse drug reaction, cause the at least one computing device to perform operations comprising:
retrieving data associated with a plurality of agents of an agent tree;
retrieving task data of each agent of the plurality of agents;
analyzing the retrieved data associated with the plurality of agents and the task data of each agent of the plurality of agents for refining task prompts and delegation of one or more tasks to an agent of the plurality of agents;
generating based upon the analyzing, a list of optimum parameters and interaction of the agent with other agent of the plurality of agents; and
generating using the list of optimum parameters, one or more backstories for training the agent of the plurality of agents, wherein the agent is trained for automatic reporting of the adverse drug reaction.
